# EUROPEAN PATENT SPECIFICATION

(11) **EP 2 170 259 B1**
(45) Date of publication and mention of the grant of the patent: **05.09.2018**
(21) Application number: 08774551.9
(22) Date of filing: 30.06.2008
(51) Int. Cl.: A61K 8/41, A61K 8/49, A61K 8/60, A61Q 19/06, A61K 31/132, A61P 3/04, A61P 17/00

(54) **SLIMMING COMPOSITION**
SCHLANKHEITSZUSAMMENSETZUNG
COMPOSITION D'AMAIGRISSEMENT

(30) Priority: 28.06.2007 FR 0756112; 08.01.2008 US 10332 P; 12.03.2008 FR 0801348
(43) Date of publication of application: 07.04.2010
(60) Divisional application: 12186009.2
(73) Proprietor: BASF Beauty Care Solutions France SAS, 69007 Lyon (FR)
(72) Inventor: BONNET, Isabelle, F-69007 Lyon (FR); GODARD, Nathalie, F-69610 Aveize (FR); PERRIER, Eric, F-38138 Les Cotes D'Arey (FR)
(74) Representative: Mendelsohn, Isabelle M. N.
(86) International application number: PCT/EP2008/058398
(87) International publication number: WO 2009/000935

(56) References cited:
- EP-A- 0 067 356
- WO-A-2006/048671
- US-A1- 2003 143 713
- US-A1- 2004 146 539
- US-A1- 2006 193 814
- YUAN ET AL: "Antioxidant activity and cytoprotective effect of kappa-carrageenan oligosaccharides and their different derivatives" 1 March 2006 (2006-03-01), BIOORGANIC & MEDICINAL CHEMISTRY LETTERS, OXFORD, GB, PAGE(S) 1329-1334 , XP005263946 ISSN: 0960-894X the whole document
- GUIBET ET AL: "Complete assignment of <1>H and <13>C NMR spectra of Gigartina skottsbergii lambda-carrageenan using carrabiose oligosaccharides prepared by enzymatic hydrolysis" 14 August 2006 (2006-08-14), CARBOHYDRATE RESEARCH, ELSEVIER SCIENTIFIC PUBLISHING COMPANY. AMSTERDAM, NL, PAGE(S) 1859-1869 , XP005502826 ISSN: 0008-6215 the whole document

## Description

The invention relates to active slimming ingredients and an industrial process to prepare them.

### STATE OF THE ART

An entire field of cosmetics and pharmacy is involved with active slimming ingredients. Various active ingredients in this field are intended to overcome problems with inesthetic tissues like "orange peel skin" caused by adipocytes overloaded with fatty acids.

Spermine and spermidine are polyamines which are present in adipose tissue and especially in adipocytes in average concentrations of 0.16 and 0.30 nmol/mg respectively (Pedersen et al., 1989, Mol. Cell. Endocrinol, 62, 161-166).

*In vitro,* these polyamines have been demonstrated to stimulate three major enzymes of the lipogenesis process, a name given to the process of storing fatty acids in the form of triglycerides in the adipocytes.

These enzymes are:
- sn-glycerol-3-phosphate acyltransferase or GPAT;
- 1,2-diacyl-sn-glycerol acyltransferase or DGAT;
- Mg²⁺ phosphatidate phosphohydrolase or MGPPH.

In 1996, Jamdar et al. (1966, Enzyme protein, 49, 222-230) discovered a correlation in obese rats between the concentrations of spermine and spermidine contained in adipose tissues and an increase in body fats. This increase correlates to a strong stimulation of the enzymes involved in the lipogenesis process, such as GPAT, DGAT or MGPPH, which demonstrates *in vivo* the significant involvement of polyamines in the fat storing process.

A fourth enzyme, lipoprotein lipase (or LPL) in charge of transporting fatty acids in the bloodstream to the adipocytes, also shows stimulation of its activity in the presence of spermine and spermidine (Giudicelli et al., 1976, FEBS Lett., 62, 1, 74-76).

Finally, spermine and spermidine have been identified as acting as "insulin-iike" factors on the metabolism of adipocytes, meaning that these polyamines facilitate glucose transport, improve its conversion into triglycerides via stimulation of pyruvate dehydrogenase (Rutter et al., 1992, Biochem. J., 285, 435-439) and inhibit adipocytes lypolytic activities (Olefsky et al., 1979, Horm. Metab. Res., 11, 209-213 ; Lockwood et al., J. Biol. Chem., 1974, 249, 24, 7717-7722; Richelsen et al., Biochem. J., 1989, 261, 2, 661-665).

Finally, Bethell et al., (1981, Biochim. Biophys. Res. Commun., 102, 1, 272-278) have shown that differentiation of fibroblasts into adipocytes is accompanied by a significant increase in the spermidine rate. This polyamine seems therefore to play an important role in the process of transforming fibroblasts into adipocytes.

Spermine and spermidine therefore foster the storage of fats in the adipocytes (via stimulation of the enzymes involved in the lipogenesis process) and inhibit the liberation of these fats (via inhibition of lipolysis). If these two polyamines are blocked, the expected effect would be inhibition of the lipogenesis process and stimulation of lipolysis, hence an overall slimming effect.

To date, no active ingredient is known to act on spermine and/or spermidine.

### PURPOSES OF THE INVENTION

The main objective of the invention is to solve the new technical problem which consists in supplying an industrial process for the preparation of active slimming ingredients.

This invention is especially intended to solve the new technical problem consisting in acting on spermine and/or spermidine, which are molecules involved in lipogenesis/lipolysis, especially at the adipocytes level,

This invention is intended to solve the above-described technical problems in an industrial, reproducible and reliable manner, at the lowest cost, especially in the cosmetic, nutriceutical and/or pharmaceutical fields.

### DESCRIPTION OF THE INVENTION

It was surprisingly discovered that compositions comprising one or several active ingredients capable of trapping spermine and/or spermidine can be prepared, and thus act on lipogenesis and/or lipolysis at the adipocytes level, especially in human beings.

Thus, this invention relates to substances which trap spermine and/or spermidine,

Trapping of spermine and/or spermidine enables reduction of lipogenesis and therefore a decrease in the storage of fats, but also an increase in lipolysis thus fostering the breakdown of fats, which thereby induces a twofold action towards a slimming effect.

By "trapping of spermine and/or spermidine" the inventors mean: the association of spermine and/or spermidine with a compound called a "substance which traps spermine and/or spermidine" by any means, and preferably by complexation.

Advantageously, a substance trapping spermine and/or spermidine in an efficient manner is a substance enabling inhibition of at least 50% of lipogenesis and/or stimulation of at least 50% of lipolysis, as tested on normal human adipocytes in suspension.

Inhibition of lipogenesis is understood to be the decrease in storage of fats (lipids) in the adipocytes. Stimulation of lipolysis is understood to be an increase in the concentration of non esterified fatty acids and released from adipocytes.

Among the screened potentially active substances, sulfated polysaccharides belonging to the family of sulfated galactans, as for example carrageenans or agars, have shown unexpected effectiveness.

Specifically, the sulfated polysaccharides having the best properties are the structures containing a maximum of 20 saccharidic units, and preferably a maximum of 10 saccharidic units.

These compounds can be advantageously prepared by hydrolysis of one or several sulfated polysaccharides.

Among these preferred polysaccharides or oligosaccharides, the kappa carrageenans are preferred for better results.

Two methods are advantageously used for the hydrolysis of carrageenans:
▪ Enzymatic hydrolysis done with carrageenases extracted from *Pseudomonas carrageenovora* (Knutsen et al., 2001, carbohydr. res., 331, 101-106)
▪ Chemical hydrolysis: basic hydrolysis is generally slow and can result in ring openings. A hot reaction in the presence of hydrochloric acid (Ekström et al., 1983, carbohydr. Res. 116, 89-94) or sulfuric acid (Rochas et al., 1981, Polym. Bulletin. 5, 81-86) is preferred.
The oligosaccharides produced by the hydrolysis of carrageenans are composed of chains of carrabiose units, neocarrabiose units and neocarratetraose units. (cf. formula 1).

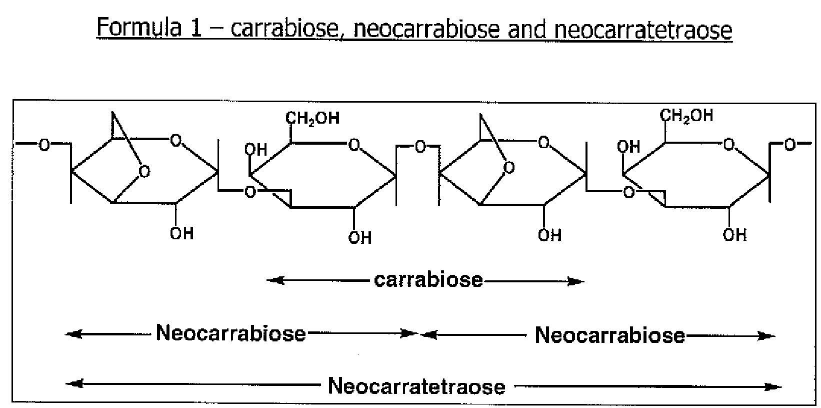

Generally, it is acknowledged that enzymatic hydrolysis of the carrageenans produce oligosaccharides comprising neocarrabiose units and that acid hydrolysis enable us to obtain oligosaccharides based on the carrabiose units (Kono et al., US Patent 4,748,032).

Advantageously, this invention relates to an improved hydrolysis process comprising introduction into a solution of at least one carrageenan, preferably for a sufficient duration and at a sufficient temperature to dissolve the carrageenans in the solvent medium, which is preferably water. The temperature is advantageously higher than 30°C.

The hydrolysis process advantageously comprises hydrolysis in an acid medium, by using for example a mineral acid such as hydrochloric acid. This hydrolysis is performed for a sufficient duration and at a sufficient temperature to hydrolyze the carrageenan into oligosaccharides. The pH of the reaction is advantageously less than 3, and preferably less than 2. It is generally preferable to perform hydrolysis of a carrageenan by an acid, such as a mineral acid like hydrochloric acid, for a duration of more than 5 hours, and preferably more than 10 hours, and even more preferably for a duration of more than 15 hours.

The reaction is advantageously stopped by the addition of a basic solution, by using for example a mineral base such as sodium hydroxide. The final pH of the preparation is generally from 3.5 to 5.

The inventors have preferentially chosen an acidic hydrolysis process from kappa-carrageenan. This process comprises an innovative variation prepared by the inventors in such a way as to obtain an optimal amount of kappa carrabiose units (cf. formula 2) comprising a beta-1.4 bond between the two saccharide units. However, this invention is not limited to such a process. Thus another hydrolysis variation is used from iota and/or lambda carrageenan.

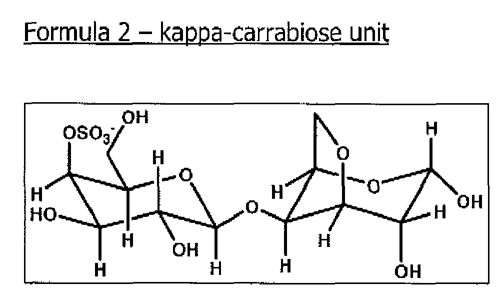

According to the nature and the concentration of the acid, the temperature and the reaction time, the oligosaccharide solutions which are obtained present different concentrations of carrabioses and neocarratetraoses.

In the present invention, the oligosaccharide solutions which present a capability for trapping spermine and spermidine have a polymerization degree , or dp, between 2 (dp 1 = one carrabiose unit) and 20, preferably between 2 and 10, and still even more preferably a maximum concentration of 2 dp units (or carrabioses).

Advantageously, following the hydrolysis process (chemical or enzymatic), all the insoluble parts are removed from the mixture, by centrifugation or by filtration.

If necessary, the oligosaccharide solutions can be purified for example by discoloration on active charcoal or ion exchange resins, or separated for example on membranes or gel chromatography.

The solutions can be transformed for example into powder especially by freeze-drying, atomization, crystallization, etc.

The solution of sulfated oligosaccharides is composed advantageously of at least 0.1% carrabiose, advantageously at least 0.2% and preferably at least 0.5% (p/p), and generally does not comprise more than 10% (p/p) carrabiose, with respect to the weight of the total solution. The solution of sulfated oligosaccharides preferably comprises less than 1% of carrabioses. However, the solution of sulfated oligosaccharides can be freeze dried, thus enabling the production of a higher percentage of carrabiose as for example up to 30%, and even 40 % (p/p) or more of the freeze dried composition.

This solution of sulfated oligosaccharides, which is considered in the present invention as being the active ingredient, can be diluted in the final composition. Typically, the solution of sulfated oligosaccharides is used at a concentration between 0.01 and 10%, preferably between 0.1 and 5% in weight of the final composition. Thus the final composition comprises a percentage of carrabiose generally between 1.10⁻⁵ and 10%, preferably between 2.10⁻⁵ and 0.1% by weight.

The solution of sulfated oligosaccharides advantageously presents a pH below 7, and preferably between 3.5 and 5.

Solution of oligosaccharides is understood to be a liquid composition, possibly in the form of a gel or hydrogel, which is water based, but not exclusively. This solution can be an aqueous solution, possibly comprising different types of ions or solutions, and even insoluble or partially soluble compounds. This solution can also be a solution comprising solvents other than water, in which the sulfated oligosaccharides are soluble. The solvent is advantageously acceptable on a dermatological basis.

The compounds according to the present invention are used for the preparation of compositions, especially topical or orally administrated, and especially in the form of cosmetic, nutraceutical, dermo-pharmaceutical or pharmaceutical compositions. Thus, for these compositions, the excipient contains for example at least one compound chosen from a group consisting of preservatives, emollients, emulsifiers, surfactants, moisturizers, thickeners, conditioners, matte finishing agents, stabilizers, antioxidants, texturizers, shining agents, film forming agents, solubilizing agents, pigments, dyes, perfumes and sunscreens. These excipients are preferably chosen from a group consisting of amino acids and their derivatives, polyglycerols, esters, polymers and cellulose derivatives, lanolin derivatives, phospholipids, lactoferrins, lactoperoxidases, sucrose based stabilizers, E vitamins and their derivatives, natural and synthetic waxes, vegetable oils, triglycerides, unsaponifiable matter, phytosterols, plant esters, silicones and their derivatives, protein hydrolysates, Jojoba oils and their derivatives, lipo/hydrosoluble esters, betains, aminoxides, plant extracts, esters of saccharose, titanium dioxide, glycines, parabens, and even preferably the group consisting of butylene glycol, steareth-2, stereath-21, glycol-15 stearyl ether, cetearyl alcohol, phenoxyethanol, methylparaben, ethylparaben, propylparaben, butylparaben, butylene glycol, natural tocopherols, glycerin, dihydroxycetyl sodium phosphate, isopropyl hydroxycetyl ether, glycol stearate, triisononanoin, octyl cocoate, polyacrylamide, isoparaffin, laureth-7, a carbomer, propylene glycol, glycerol, bisabolol, a dimethicone, sodium hydroxide, PEG 30-dipolyhydroxysterate, capric/caprylic triglycerides, cetearyl octanoate, dibutyl adipate, grape seed oil, jojoba oil, magnesium sulphate, EDTA, a cyclomethicone, xanthan gum, citric acid, sodium lauryl sulphate, mineral waxes and mineral oils, isostearyl isostearate, dipelargonate of propylene glycol, isostearate of propylene glycol, PEG 8 beeswax, glycerides of hydrogenated palm oil, lanolin oil, sesame oil, cetyl lactate, lanolin alcohol, castor oil, titanium dioxide, lactose, saccharose, low density polyethylene, and isotonic saline solution.

Advantageously, the aforementioned compositions are formulated under a form chosen from the group consisting of a solution, aqueous or oil-based, a cream or an aqueous gel, or an oil-based gel, especially in a jar or tube, especially in a shower gel, a shampoo, a lotion, an emulsion, a micro- emulsion or a nano-emulsion, especially oil-in-water or water-in-oil or multiple or silicone; a lotion, especially in a glass bottle, in plastic or in a pump dispenser or in an aerosol; a vial; a liquid soap; a dermatological bar; an ointment; a foam; an anhydrous product, preferably liquid, paste or solid, for example in the form of a stick notably a lipstick.

The terms "topical application", as used herein, mean applying or spraying the composition according to this invention on the skin surface and/or on the surface of mucous membranes.

The terms "dermatologically acceptable", as used herein, mean that the composition or the components of the composition are adapted to be used in contact with human skin without unwarranted toxicity, incompatibility, instability, allergic response, or their equivalents.

Numerous cosmetically active ingredients are known by man skilled in the art to improve the health and/or the physical appearance of the skin. The man skilled in the art knows how to formulate the cosmetic or dermatological compositions to obtain the best effects. Furthermore, the compounds described in the present invention can have a synergetic effect when combined one with the others. These combinations are also covered by this invention. The CFTA Cosmetic Ingredient Handbook Second Edition (1992) describes various cosmetic and pharmaceutical field frequently utilized in the cosmetic and pharmaceutical industry, which are particularly adapted to topical application. Some examples of these classes of ingredients include, but are not limited to, the following compounds: abrasive, absorbent, esthetic compounds such as perfumes, pigments, dyes, essential oils, astringents (for example, clove oil, menthol, camphor, eucalyptus oil, eugenol, menthyl lactate, hamelis distillate), anti-acne agents, anti-floculant agents, non foaming agents, anti-microbiotic agents (for example: iodopropyl butylcarbamate), antioxidants, bonding agents, buffering agents, expanding agents, chelating agents, additives, biocide agents, denaturing agents, external analgesics, film forming materials, polymers, opacifiers, pH adjusters, reducing agents, discoloring or clarifying agents (for example, hydroquinone, kojic acid, ascorbic acid, magnesium ascorbyl phosphate, ascorbyl glucosamine), conditioning agents (for example: hygroscopic agents), soothing agents for the skin and/or scaring agents (for example: panthenol and its derivatives, for example: ethyl panthenol), aloe vera, pantothenic acid and its derivatives, allantoine, bisabolol, and dipotassium of glycyrrhizinate), thickeners, vitamins and their derivatives or equivalents.

An advantageous embodiment of the present invention comprises the addition of at least another slimming active ingredient, notably chosen from a group consisting of inhibiting agent of phosphodiesterase enzyme, activating agent of adenylate cyclase, cyclic AMP or lipolytic derivatives and/or mixtures thereof.

Preferably, the present invention comprises the addition of at least caffeine and/or forskolin and/or theophylline and/or theobromine.

Different plant extracts generally used by formulating operators in slimming formulations such as for example root extract of Coleus Forskohlii, bark extract of cecropia obtuse or sea lettuce (uva lactuca) can also be used as slimming active ingredient.

It is particularly advantageous to combine the active ingredients of this invention with caffeine. It was discovered indeed, that these ingredients have complementary acting mechanisms. Preferably, caffeine, possibly in the form of derivatives and/or salts of caffeine acid notably a salt of caffeine carboxylic acid such as those described in the French patent application FR2624010 (Pierre Fabre Cosmetique), will be administered in combination with an active ingredient according to the invention, at a dose between 0,001% and 10% by weight of total composition, preferably between 1 and 5 %.

Combination of active ingredients according to the present invention with at least one activating agent of adenylate cyclase, in particular forskolin or plant extract containing forskolin is particularly advantageous.

Preferably, forskolin, possibly as Coleus Forskohlii extract or Plectanthus barbatus extract, will be administrated combinated with active ingredients according to the present invention at dosage between 0,001% and 1%, preferably between 0,05 to 0,25% by weight of the total composition.
It is also particularly advantageous to combine the active ingredients according to present invention with one compound chosen among:
- glycosaminoglycans (GAG) stimulating agents, and/or elastin stimulating agents and/or collagen stimulating agents, in particular retinol and/or vitamin C as well as derivatives thereof, tetrapeptides containing between 50 to 500ppm of palmitoyl-Gly-Gln-pro-Arg such as those commercially available under tradename Matrixyl™ from Sederma, France;
- lipoprotein lipase inhibiting agent such as those described in patent US2003086949 (Coletica) in particular Peru liana extract (Uncaria tomentosa) or an extract of St John's wort;
- active components imitating the effect of beta endorphin in order to improve the barrier function of the skin, like for example those cited in patent application US 2006069032, extract of cocoa bean hull;
- active components stimulating the synthesis of beta endorphin in order to give a sensation of well-being, for example Tephroline, from the plant tephrosia purpurea (Soliance);
- active components stimulating cellular proliferation and/or cellular differentiation, intended to have anti-aging activity, such as the following molecules: NGF, α-MSH, β endorphin, or derivatives, for example those described in patent application FR 2857874, P3-endorphin;
   - active components protecting Fibroblast Growth Factor against degradation, notably FGF2 such as vegetal extract described in patent application filed by Applicant published under GB244036 in particular Hibiscus Abelmoscus extract;
- active components stimulating activity and/or proliferation of fibroblasts such as a fermented peptide of soy like a product marketed by Applicant under the tradename Phytokine™, optionally in combination with a Hibiscus Abelmoscus extract;
- active components stimulating Hyaluronase synthase, notably Hyaluronase synthase 2 as described in patent FR2893252;
- active components stimulating activity and/or synthesis of Lysyl Oxidase like LOXL such as components described in patent number FR2855968 in particular dill extract for stimulation of elastic fibers;
- active components with anti-inflammatory properties such as those inhibiting PLA2, and in particular components described in patent FR2847267 and notably an extract of pueraria lobata roots (Inhipase®);
- active substances permitting a change in the color of the skin, such as active components for skin lightening and/or whitening;
- active substances with draining properties, like hesperitine laurate (Flavagrum®), or quercitine caprylate(Flavenger®).

The invention covers a composition comprising (i) a preparation of sulfated oligosaccharides trapping spermine and/or spermidine, and preferably as defined more particularly in the whole description and examples of the invention, and (ii) at least one of the group consisting of caffeine, theophylline, theobromine, and forskolin, or derivatives and/or salts thereof.

A derivative is a compound that is formed from a similar compound or a compound that can be imagined to arise from another compound, if one atom is replaced with another atom or group of atoms. This definition is common in organic chemistry.

The present invention covers a combination of (i) a preparation of sulfated oligosaccharides trapping spermine and/or spermidine, and preferably as defined more particularly in the whole description and examples of the invention, and (ii) at least one of the group consisting of caffeine, theophylline, theobromine, and forskolin, or derivatives and/or salts thereof, used as a slimming active ingredient.

This invention thus relates to a method for a slimming care comprising the use of at least one active ingredient, or of a cosmetic composition comprising such an active ingredient, as referenced above or hereafter. This cosmetic care includes the topical application of the active ingredient on the cutaneous areas to be treated. This cosmetic care can be performed via the administration of a nutraceutical composition (oral administration). The cutaneous area to be treated in a subject is advantageously an area comprising adipocytes with more fatty acids than the rest of adipocytes of the cutaneous tissue.

These areas are typically orange peel skin areas.

This invention also relates to a method of therapeutic treatment to reduce the amount of lipids stored by the adipocytes, including especially the administration or the topical application of at least one active ingredient, or of one cosmetic composition comprising such an active ingredient, as referenced above or hereafter. The treatment advantageously combines both a decrease in lipogenesis and an increase in lipolysis. This treatment may be localized on the cutaneous areas to be treated.

Other objectives, characteristics and advantages of the invention will be clearly apparent for the man skilled in the art when reading the explanatory description which refers to examples which are given only for the purposes of illustration and which in no way are intended to limit the scope of the invention.

The examples are an integral part of this invention and any characteristic which seems new with respect to any previous state of the art taken from the description in its entirety, including the examples, is an integral part of the invention in its function and in its generality.

Thus, each example is general in scope.

Furthermore, in the examples, all percentages are given in weight, unless otherwise indicated, and the temperature is expressed in Celsius degree unless otherwise indicated, and pressure is atmospheric pressure, unless otherwise indicated.

### EXAMPLES

### Example 1: Preparation of the oligosaccharides

38.5 mL of pure laboratory water was heated to 60°C.

0.49 g of sodium chloride was added, as well as 1.67 g of kappa-carrageenans.

The solution was left under agitation, at 60°C for 25 minutes. The hydrolysis reaction was initialized by the addition of 8.33 g of a solution of a 1 N of hydrochloric acid and the mixture was maintained at a temperature of 60°C during the entire process. At the time of desired hydrolysis, the reaction was then stopped by the addition of 9 mL of a solution of 1 N sodium hydroxide. The pH of the preparation was therefore between 4 and 4.5.

The solution was then filtered through a 500 nm filter and then preservatives, as well as a gelling agent, can be added to the preparation.

In most of the following examples, the experiments were performed on the filtrate (without a preservative and without gelling agent).

### Example 2: Evolution of the composition of the oligosaccharides preparations with regard to hydrolysis time.

Sulfated oligosaccharide solutions were prepared according to example 1, with hydrolysis times of 2, 5 and 16 hours.

These solutions were then analyzed by HPLC (Waters Alliance 2685, column TSK gel 2000 PWXL) coupled with a refractometer detector.

A standard range of glucose was analyzed in parallel, which thus enables to know the composition of our mixture in terms of carrabiose and neocarratetraose.

The results, presented in table 1, show that the longer is the hydrolysis time, the more significant the concentration of carrabiose will be.

**Table 1**

| **Hydrolysis time (hours)** | **% carrabiose** | **% neocarratetraose** |
|---|---|---|
| 2 | 0.106 | 0.228 |
| 5 | 0.365 | 0.180 |
| 16 | 0.890 | 0.043 |

% of carrabiose or neocarratetraose indicated in % by weight, based on the total filtrate weight, said filtrate being adjusted at a pH between 4 and 4,5.

### Example 3: In vitro demonstration of the effectiveness of oligosaccharides on the trapping of spermine and spermidine

### Principle:

If spermine and spermidine are put in the presence of deoxyribonucleic acid (DNA), precipitation of DNA is then observed and the optic density of the mixture, read at 600 mn, increases very significantly.

If a compound which "traps" spermine or spermidine is added beforehand to the solution of said polyamine, no precipitation appears when the DNA solution is added.

We can then calculate a percentage of protection provided by the tested active ingredient in terms of the DNA precipitation induced by the polyamines. Protocol:
0.4 g of fish DNA (Sigma) is added to 160 g of a pH6 phosphate buffer (0.06 M potassium phosphate and 0.001 M sodium phosphate).

10 µL of an aqueous solution of 50 mM of spermine (Sigma) is distributed into each well of a 96-well plate:
Then the following is distributed:
- 90 µL of a solution of sulfated oligosaccharides (active ingredient) or of an aqueous solution of 150 mM of adenosine triphosphate (or ATP, Sigma)
- or 90 µL of pure laboratory water (in the "control" wells or "100%" wells)

The plate is left under agitation for 5 minutes at room temperature, then 100 µL of the DNA solution is added to all of the wells.

After 5 seconds of manual agitation, the optic density of the well is read at 600 nm.

The percentage of protection provided by the active ingredient in terms of the precipitation of the DNA in the presence of spermine is calculated as follows:

### % protection = 100 - [(OD _{Active or ATP} / OD _{100%}) x 100]

By **"OD _{Active or ATP}"** we mean the optic density of the well containing DNA, spermine or spermidine, and the Active Ingredient or the ATP after reaction.

"OD 100%" means the optic density of the well containing DNA and spermine or spermidine, after precipitation.

The more significant the protection percentage is, the greater is the "trapping" of spermine by the active ingredient and the lesser is the DNA precipitation.

The same experiment can be performed by replacing the solution of 50 mM spermine with an aqueous solution of 300 mM spermidine (Sigma),

Oligosaccharides solutions were produced according to example 1, with 2, 5 and 16 hours of hydrolysis time.

After dilution at 10% in pure laboratory water, the different solutions were tested.

The results are given in table 2 below:

**Table 2**

| **Hydrolysis time** | **% spermine protection** | **% spermidine protection** |
|---|---|---|
| 2 | 3.9 | 29.9 |
| 5 | 4.5 | 28.2 |
| 16 | 10.3 | 34.3 |

The longer the hydrolysis time is, the richer the oligosaccharides solution is in carrabiose and the bigger is the trapping action of the spermine and spermidine.

Thus active ingredients containing at least 0.2% of carrabiose are preferred, and even more preferred, at least 0.5% by weight of the solution of sulfated oligosaccharides.

### Example 4: Comparison of the carrabiose rate after hydrolysis of various sulfated galactans

Solutions of sulfated oligosaccharides were prepared using kappa, iota and lambda carrageenans and agar according to example 1, with a hydrolysis time of 16 hours.

The extracts were then analyzed by HPLC according to the technique described in example 2, with the exception of agar hydrolysates which couldn't be analyzed under the same conditions (agarobiose units).

After dilution at 3% in pure laboratory water, a trapping test of the spermidine was performed, according to the protocol described in example 3.

**Table 3:**

| **Hydrolyzed galactans** | **% Trapping** | **% Carrabiose** | **% Neocarratetraose** |
|---|---|---|---|
| Kappa-carrageenans | 16.1% | 0.878 | 0.155 |
| Iota-carrageenans | 13.1% | 0.292 | 0.344 |
| Lamda-carrageenans | 13.5% | 0.179 | 0.197 |
| agar | 8.9% | Not applicable | Not applicable |

The carrageenan hydrolysates (kappa, iota and lambda) show the best effectiveness in terms of trapping spermidine. This trapping is correlated at a percentage of carrabioses in the product.

### Example 5: Demonstration of the effectiveness of oligosaccharides on the inhibition of lipogenesis in normal human adipocytes.

The *ex vivo* inhibition of lipogenesis was evaluated in suspensions of human adipocytes which were isolated from a sampling taken during an abdominal plasty performed on a 42 year old woman.

The adipocytes suspension was incubated for one hour at 37°C with 3 or 5% of a solution of sulfated oligosaccharides prepared according to example 1 (hydrolysis time: 16 hours), or with a standard study solution (1mM theophylline or caffeine), on in the absence of these solutions (control).

Each assay was done in triplicate.

A radioactive marker [2-¹⁴C] acetate was then added to the various assays at a concentration of 2.5 µCi/mL.

After 4 hours of incubation at 37°C, the lipids were extracted by a methanol/chloroform/water mixture and then dehydrated. The radioactivity, expressed in counts per minute (cpm), is then quantified through liquid scintigraphy (LKB 1210 Rackbeta).

The results are given in table 4 below.

The statistical analysis was performed according to the Dunnett's multiple comparison test.

**Table 4**

| **Treatment** | **Concentration in water** | **cpm** | **Standard deviation** | **% Control** | **p** |
|---|---|---|---|---|---|
| Control | - | 472253 | 4474 | 100 | - |
| Theophylline | 1 mM | 27225 | 6195 | 6 | P<0.01 |
| Caffeine | 1 mM | 20741 | 2932 | 4 | P<0.01 |
| Sulfated Oligosaccharides | 5% | 109647 | 7064 | 23 | P<0.01 |
| | 3% | 97139 | 9691 | 21 | P<0.01 |

According to the experimental conditions, the solution of sulfated oligosaccharides tested at 3% to 5% significantly inhibits lipogenesis and in a dose-dependent manner.

### Example 6: Demonstration of the effectiveness of the preparation on the stimulation of lipolysis in normal human adipocytes

The lipolysis stimulation activity was evaluated in suspensions of human adipocytes isolated from a sample taken during an abdominal plasty performed on a 34 year old woman.

The adipocytes suspension was incubated for 2 hours at 37°C with 3 or 5% of a solution of sulfated oligosaccharides prepared according to example 1 (16 hours of hydrolysis time) in percentage of total weight of the culture, or with standard study solutions (1mM theophylline or caffeine) or in the absence of solutions (control). Each assay was performed 6 times.

At the end of the incubation period, the Non Esterified Fatty Acids (NEFA) which were present in the incubation mediums were measured with commercially available dosage kits (OXOID 46 551).

The results are given in table 5 below.

**Table 5**

| **Treatment** | **Conc**. | **NEFA in µM** | **Standard deviation** | **% Control** | **p** |
|---|---|---|---|---|---|
| Control | - | 96 | 6 | 100 | |
| Theophylline | 1 mM | 537 | 45 | 560 | P<0.01 |
| Caffeine | 1 mM | 525 | 40 | 547 | P<0.01 |
| Sulfated Oligosaccharides | 5% | 200 | 10 | 209 | P<0.01 |
| | 3% | 180 | 12 | 187 | P<0.01 |

According to the experimental conditions, the solution of sulfated oligosaccharides tested at 3% and 5% significantly stimulates lipolysis and in a dose-dependent manner.

### Example 7: Demonstration of a slimming effect of the oligosaccharides according to the invention in combination with caffeine

The slimming effectiveness of a hydro-alcohol gel containing a mixture of "3% caffeine + 3% of a solution of sulfated oligosaccharides prepared according to example 1 (16 hours of hydrolysis time)" was compared to a hydro-alcohol gel containing 3% caffeine.

The products were applied to the thighs of healthy volunteers and the slimming effect was evaluated over an 8 weeks period by measuring in centimeters.

In practice, 25 volunteers aged 21 to 49 were selected and tested according to specific criteria (Body Mass Index, presence of cellulite). The weight of each volunteer was monitored during the entire study and couldn't vary from ± 2 kg. The products were applied according to a randomization of right thigh/left thigh: one thigh treated by the association (caffeine + oligosaccharides solution) and one thigh treated by caffeine. The gels were applied twice a day for 8 consecutive weeks, using small circular movements until the entire product was penetrated.

The chosen formulation for this study was a hydro-alcohol gel on the basis of a ratio of 75% water to 25% denatured alcohol.

Measurement in centimeters of the thighs circumference was done at the middle of the volunteers' thighs using a tape measure applied without compression by the same technician for the entire study.

A laser range-finding system for the measurement zones was used to ensure that same positioning for each thigh evaluated was reproduced and precise.

The measurements were performed before application of the products (T0) and then after 14 days, 1 month and 2 months.
The results of this study are in table 6 below.

**Table 6:**

| Time | Caffeine | Caffeine + sulfated oligosaccharides | Significance of results ("Caffeine + sulfated oligosaccharides" versus "caffeine") |
|---|---|---|---|
| 14 days | 0 cm | -0.1 cm | Not significant |
| 28 days | -0.1 cm | -0.4 cm | p <0.001 |
| 56 days | -0.3 cm | -0.6 cm | p < 0.01 |

After only 14 treatment days, a decrease in the circumference of the volunteers" thighs having received applications of the gel containing the caffeine and the sulfated oligosaccharides was observed. It should be noted that the gel containing caffeine alone had no slimming effect.

This excellent effectiveness due to the combination of caffeine and sulfated oligosaccharides is even more evident after 28 and 56 days of treatment.

Indeed, at 56 days, the average decrease of the circumference of the thigh is 0.6 cm for the volunteers using the caffeine+oligosaccharide gel, while the decrease is only 0.3 cm for the volunteers who applied the caffeine gel (significant difference, p<0.01).

### Example 8: Use of the products of the invention in cosmetic or pharmaceutical formulations of the type "oil emulsion in water"

**Formulation 8a:**

| | | |
|---|---|---|
| A | Water | qsp 100 |
| | Butylene Glycol | 2 |
| | Glycerin | 3 |
| | Sodium Dihydroxycetyl Phosphate, Isopropyl Hydroxycetyl Ether | 2 |
| | | |
| B | Glycol Stearate SE | 14 |
| | Triisononaoin | 5 |
| | Octyl Cocoate | 6 |
| | | |
| C | Butylene Glycol, Methylparaben, Ethylparaben, Propylparaben, pH adjusted to 5,5 | 2 |
| | | |
| D | Products of the invention | 0.01 - 10 % |

**Formulation 8b:**

| | | |
|---|---|---|
| A | Water | qsp 100 |
| | Butylene Glycol | 2 |
| | Glycerin | 3 |
| | Polyacrylamide, Isoparaffin, Laureth-7 | 2.8 |
| | | |
| B | Butylene Glycol, Methylparaben, | 2 |
| | Ethylparaben, Propylparaben ; | |
| | Phenoxyethanol, Methylparaben, Propylparaben, Butylparaben, Ethylparaben | 2 |
| | Butylene Glycol | 0.5 |
| | | |
| D | Products of the invention | 0.01 - 10 % |

**Formulation 8c:**

| | | |
|---|---|---|
| A | Carbomer | 0.50 |
| | Propylene Glycol | 3 |
| | Glycerol | 5 |
| | Eau | qsp 100 |
| | | |
| B | Octyl Cocoate | 5 |
| | Bisabolol | 0.30 |
| | Dimethicone | 0.30 |
| | | |
| C | Sodium Hydroxide | 1.60 |
| | | |
| D | Phenoxyethanol, Methylparaben, Propylparaben, Butylparaben, Ethylparaben | 0.50 |
| E | Perfume | 0.30 |
| | | |
| F | Products of the invention | 0.01 - 10 % |

### Example 9: Use of the products of the invention in a formulation of type "water in oil"

| | | |
|---|---|---|
| A | PEG 30 - dipolyhydroxystearate | 3 |
| | Capric Triglycerides | 3 |
| | Cetearyl Octanoate | 4 |
| | Dibutyl Adipate | 3 |
| | Grape Seed Oil | 1.5 |
| | Jojoba Oil | 1.5 |
| | Phenoxyethanol, Methylparaben, Propylparaben, Butylparaben, Ethylparaben | 0.5 |
| | | |
| B | Glycerin | 3 |
| | Butylene Glycol | 3 |
| | Magnesium Sulfate | 0.5 |
| | EDTA | 0.05 |
| | Water | qsp 100 |
| | | |
| C | Cyclomethicone | 1 |
| | Dimethicone | 1 |
| | | |
| D | Perfume | 0.3 |
| | | |
| E | Products of the invention | 0.01 - 10 % |

### Example 10: Use of products of the invention in a formulation of type "shampoo or shower gel"

| | | |
|---|---|---|
| A | Xantham Gum | 0.8 |
| | Water | qsp 100 |
| | | |
| B | Butylene Glycol, Methylparaben, Ethylparaben, Propylparaben | 0.5 |
| | Phenoxyethanol, Methylparaben, Propylparaben, Butylparaben, Ethylparaben | 0.5 |
| | | |
| C | Citric acid | 0.8 |
| | | |
| D | Sodium Laureth Sulfate | 40.0 |
| | | |
| E | Product of the invention | 0.01 - 10 % |

### Example 11: Use of products of the invention in a formulation of aqueous gels

| | | |
|---|---|---|
| A | Water | qsp 100 |
| | Carbomer | 0.5 |
| | Butylene Glycol | 15 |
| | Phenoxyethanol, Methylparaben, Propylparaben, Butylparaben, Ethylparaben | 0.5 |
| | | |
| B | Products of the invention | 0.01 - 10 % |

### Example 12: Use of products of the invention in a formulation type "triple emulsion"

**Primary emulsion W1/O**

| | | |
|---|---|---|
| A | PEG 30 - dipolyhydroxystearate | 4 |
| | Capric Triglycerides | 7.5 |
| | Isohexadecane | 15 |
| | PPG-15 Stearyl ether | 7.5 |
| | | |
| B | Product of the invention | 0.01-10% |
| | Water | Qsp 65.3 |
| | | |
| C | Phenoxyethanol, Methylparaben, Propylparaben, Butylparaben, Ethylparaben | 0.7 |

**Secondary emulsion W1/O/W2**

| | | |
|---|---|---|
| A | Primary emulsion | 60 |
| | | |
| B | Poloxamer 407 | 2 |
| | Phenoxyethanol, Methylparaben, Propylparaben, 2-bromo-2nitropropane-1,3 diol | 0.3 |
| | water | qsp 100 |
| C | Carbomer | 15 |
| | | |
| D | Triethanolamine | PH 6.0-6.5 |

### Example 13: Preparation of pharmaceutical formulations containing the product of the invention

**Formulation 13a: preparation in tablet form**

| | | |
|---|---|---|
| A | Excipients | In g per tablet |
| | Lactose | 0.359 |
| | Saccharose | 0.240 |
| | | |
| B | Products of the invention* | 0.001-0.1 |

| | | |
|---|---|---|
| *The product of the invention is obtained, for example, according to the extraction process described in example 1 (16 hours of hydrolysis time) followed by a drying stage. | | |

**Formulation 13b: preparation of an ointment**

| | | |
|---|---|---|
| A | Excipients | |
| | Low density polyethylene | 5.5 |
| | Liquid paraffin | qsp 100 |
| | | |
| B | Products of the invention* | 0.001-0.1 |

| | | |
|---|---|---|
| *The product of the invention is obtained, for example, according to the extraction process described in example 1 (16 hours of hydrolysis time) followed by a drying stage. | | |

**Formulation 13c: preparation of an injectable formula**

| | | |
|---|---|---|
| A | Excipient | |
| | Isotonic salt solution | 5 mL |
| | | |
| | Products of the invention* | 0.001 -0.1 g |

| | | |
|---|---|---|
| *The product of the invention is obtained, for example, according to the extraction process described in example 1 (16 hours of hydrolysis time) followed by a drying stage. | | |

## Claims

1. Use of a preparation comprising sulfated oligosaccharides capable of trapping spermine and/or spermidine, as an active slimming ingredient in a non-therapeutic cosmetic composition.

2. Use, according to claim 1, wherein the oligosaccharides are composed of a maximum of 20 galactose units.

3. Use of a sulfated oligosaccharide preparation, according to any of the preceding claims, wherein the preparation of sulfated oligosaccharides is obtained by a process comprising a step of hydrolysis of a polysaccharide, preferably from sulfated galactans family, and preferably belonging to the family of carrageenans.

4. Use of a sulfated oligosaccharide preparation, according to any of the preceding claims, wherein the polysaccharide belongs to the family of kappa-carrageenans.

5. Use, according to any of the preceding claims, wherein the preparation predominantly comprises, among the present sulfated oligosaccharides, at least one oligosaccharide based on two saccharidic units (carrabiose and neocarrabiose) and possibly, at least one oligosaccharide based on four saccharidic units (neocarratetraose).

6. Use, according to any of the preceding claims, wherein the preparation of sulfated oligosaccharides is between 1.10⁻⁵ and 10% by weight of the total composition.

7. Use, according to any of the preceding claims, wherein the preparation of sulfated oligosaccharides is a solution and is used at a concentration of between 0.01 and 10% by weight of the final cosmetic composition.

8. Use, according to any of the preceding claims, wherein the preparation of sulfated oligosaccharides is a solution comprising at least 0.1% carrabiose, preferably at least 0.2% but no more than 10% (p/p) carrabiose, with respect to the weight of the total solution.

9. Use, according to any of the preceding claims, wherein the preparation of sulfated oligosaccharides is a solution of sulfated oligosaccharides comprising less than 1% of carrabiose.

10. Use, according to any of the preceding claims, wherein the composition also comprises at least one slimming active ingredient selected from the group consisting of caffeine, theophylline, theobromine, forskolin, caffeine acid or salts thereof, caffeine carboxylic acid or salts thereof, a root extract of *Coleus Forskohlii,* a bark extract of *cecropia obtuse,* and an extract of sea lettuce (*ulva lactuca*).

11. A slimming cosmetic composition comprising a preparation of sulfated oligosaccharides capable of trapping spermine and/or spermidine, as an active slimming ingredient, possibly in association with another active slimming ingredient, such as caffeine, theophylline, theobromine, forskolin, a root extract of *Coleus Forskohlii,* a bark extract of *cecropia obtuse,* an extract of sea lettuce (*ulva lactuca*), and/or mixtures thereof.

12. The cosmetic composition according to claim 11 wherein it is a nutraceutical composition.

13. Use of a preparation of sulfated oligosaccharides as defined in any one of claims 1 to 10, possibly in association with another active slimming ingredient, such as caffeine, theophylline, theobromine, forskolin, a root extract of *Coleus Forskohlii,* a bark extract of *cecropia obtuse,* an extract of sea lettuce (*ulva lactuca*), and/or mixtures thereof, for the preparation of a pharmaceutical composition with a slimming effect, especially for combating an overweight problem.

14. A non-therapeutic cosmetic method of slimming care comprising the topical application or oral administration of a cosmetic composition according to claims 11 or 12.

15. A pharmaceutical composition for use in a therapeutic treatment to reduce the amount of lipids stored by the adipocytes, by administration or topical application of a preparation of sulfated oligosaccharides capable of trapping spermine and/or spermidine as defined in any one of claims 1 to 10.

16. A composition comprising (i) a preparation of sulfated oligosaccharides capable of trapping spermine and/or spermidine, notably as defined in any one of claims 2 to 10, and (ii) at least one of the group consisting of caffeine, theophylline, theobromine, and forskolin.

17. A composition according to claim 16 wherein (ii) is caffeine and/or a salt thereof.

18. A composition according to claim 16 wherein (ii) is forskolin and/or a salt thereof.

## Patentansprüche

1. Verwendung einer Zubereitung umfassend sulfatierte Oligosaccharide, die Spermin und/oder Spermidin einschließen können, als aktiven, schlankmachenden Bestandteil in einer nicht therapeutischen kosmetischen Zusammensetzung.

2. Verwendung nach Anspruch 1, wobei die Oligosaccharide aus höchstens 20 Galactoseeinheiten bestehen.

3. Verwendung einer sulfatierten Oligosaccharidzubereitung nach irgendeinem der vorhergehenden Ansprüche, wobei die Zubereitung sulfatierter Oligosaccharide durch ein Verfahren erhalten wird, das einen Schritt der Hydrolyse eines Polysaccharids, bevorzugt aus der Familie sulfatierter Galactane und bevorzugt zur Familie von Carrageenen gehörend, umfasst.

4. Verwendung einer sulfatierten Oligosaccharidzubereitung nach irgendeinem der vorhergehenden Ansprüche, wobei das Polysaccharid zur Familie von Kappa-Carrageenen gehört.

5. Verwendung nach irgendeinem der vorhergehenden Ansprüche, wobei die Zubereitung hauptsächlich unter den vorliegenden sulfatierten Oligosacchariden mindestens ein Oligosaccharid auf der Basis von zwei saccharidischen Einheiten (Carrabiose und Neocarrabiose) und möglicherweise mindestens ein Oligosaccharid auf der Basis von vier saccharidischen Einheiten (Neocarratetraose) umfasst.

6. Verwendung nach irgendeinem der vorhergehenden Ansprüche, wobei die Zubereitung sulfatierter Oligosaccharide zwischen 1X10⁻⁵ und 10 Gew.-% der gesamten Zusammensetzung liegt.

7. Verwendung nach irgendeinem der vorhergehenden Ansprüche, wobei die Zubereitung sulfatierter Oligosaccharide eine Lösung ist und in einer Konzentration zwischen 0,01 und 10 Gew.-% der endgültigen kosmetischen Zusammensetzung verwendet wird.

8. Verwendung nach irgendeinem der vorhergehenden Ansprüche, wobei die Zubereitung sulfatierter Oligosaccharide eine Lösung ist, die mindestens 0,1 % Carrabiose, bevorzugt mindestens 0,2 %, jedoch nicht mehr als 10 % (Gew./Gew.) Carrabiose, mit Bezug auf das Gewicht der gesamten Lösung, umfasst.

9. Verwendung nach irgendeinem der vorhergehenden Ansprüche, wobei die Zubereitung sulfatierter Oligosaccharide eine Lösung sulfatierter Oligosaccharide ist, die weniger als 1 % Carrabiose umfasst.

10. Verwendung nach irgendeinem der vorhergehenden Ansprüche, wobei die Zusammensetzung auch mindestens einen schlankmachenden aktiven Bestandteil umfasst ausgewählt aus der Gruppe bestehend aus Koffein, Theophyllin, Theobromin, Forskolin, Koffeinsäure oder Salzen davon, Koffeincarbonsäure oder Salzen davon, Wurzelextrakt von Coleus Forskohlii, einem Rindenextrakt von cecropia obtuse und einem Extrakt von Meersalat (ulva lactuca).

11. Schlankmachende kosmetische Zusammensetzung umfassend eine Zubereitung sulfatierter Oligosaccharide, die Spermin und/oder Spermidin einschließen können, als aktiven, schlankmachenden Bestandteil, möglicherweise in Verbindung mit einem anderen aktiven schlankmachenden Bestandteil wie Koffein, Theophyllin, Theobromin, Forskolin, einem Wurzelextrakt von Coleus Forskohlii, einem Rindenextrakt von cecropia obtuse einem Extrakt von Meersalat (ulva lactuca) und/oder Mischungen davon.

12. Kosmetische Zusammensetzung nach Anspruch 11, wobei sie eine nutrazeutische Zusammensetzung ist.

13. Verwendung einer Zubereitung sulfatierter Oligosaccharide, wie in irgendeinem der Ansprüche 1 bis 10 definiert, möglicherweise in Verbindung mit einem anderen aktiven schlankmachenden Bestandteil wie Koffein, Theophyllin, Theobromin, Forskolin, einem Wurzelextrakt von Coleus Forskohlii, einem Rindenextrakt von cecropia obtuse, einem Extrakt von Meersalat (ulva lactuca) und/oder Mischungen davon, für die Herstellung einer pharmazeutischen Zusammensetzungen mit einer schlankmachenden Wirkung, insbesondere zum Bekämpfen eines Übergewichtproblems.

14. Nichttherapeutisches kosmetisches Verfahren zur schlankmachenden Pflege, umfassend die topische Anwendung oder orale Verabreichung einer kosmetischen Zusammensetzung nach den Ansprüchen 11 oder 12.

15. Pharmazeutische Zusammensetzung zur Verwendung bei einer therapeutischen Behandlung zum Reduzieren der Menge an Lipiden, die durch die Adipozyten gespeichert werden, durch Verabreichung oder topische Anwendung einer Zubereitung sulfatierter Oligosaccharide, die Spermin und/oder Spermidin einschließen können, wie in irgendeinem der Ansprüche 1 bis 10 definiert.

16. Zusammensetzung umfassend (i) eine Zubereitung sulfatierter Oligosaccharide, die Spermin und/oder Spermidin einschließen können, insbesondere wie in irgendeinem der Ansprüche 2 bis 10 definiert und (ii) mindestens eines aus der Gruppe bestehend aus Koffein, Theophyllin, Theobromin und Forskolin.

17. Zusammensetzung nach Anspruch16, wobei (ii) Koffein und/oder ein Salz davon ist.

18. Zusammensetzung nach Anspruch 16, wobei (ii) Forskolin und/oder ein Salz davon ist.

## Revendications

1. Utilisation d'une préparation comprenant des oligosaccharides sulfatés capable de piéger la spermine et/ou la spermidine, comme principe actif amincissant dans une composition cosmétique non thérapeutique.

2. Utilisation, selon la revendication 1, dans laquelle les oligosaccharides sont constitués d'au maximum 20 unités galactoses.

3. Utilisation d'une préparation d'oligosaccharides sulfatés, selon l'une quelconque des revendications précédentes, dans laquelle la préparation d'oligosaccharides sulfatés est obtenue par un procédé comprenant une étape d'hydrolyse d'un polysaccharide de préférence issu de la famille des galactanes sulfatés, et préférentiellement appartenant à la famille des carraghénanes.

4. Utilisation d'une préparation d'oligosaccharides sulfatés, selon l'une quelconque des revendications précédentes, dans laquelle le polysaccharide appartient à la famille des kappa-carraghénanes

5. Utilisation, selon l'une quelconque des revendications précédentes, dans laquelle la préparation comprend majoritairement parmi les oligosaccharides sulfatés présents au moins un oligosaccharide basé sur deux unités saccharidiques (carrabiose et néocarrabiose) et éventuellement au moins un oligosaccharide basé sur quatre unités saccharidiques (néocarratétraose).

6. Utilisation, selon l'une quelconque des revendications précédentes, dans laquelle la préparation d'oligosaccharides sulfatés est comprise entre 1.10⁻⁵ et 10% en poids de la composition totale.

7. Utilisation, selon l'une quelconque des revendications précédentes, dans laquelle la préparation d'oligosaccharides sulfatés est une solution et est utilisée à une concentration comprise entre 0.01 et 10% en poids de la composition cosmétique finale.

8. Utilisation, selon l'une quelconque des revendications précédentes, dans laquelle la préparation d'oligosaccharides sulfatés est une solution comprenant au moins 0.1% de carrabioses et avantageusement au moins 0.2% et pas plus de 10% (p/p) de carrabioses, par rapport au poids de la solution totale.

9. Utilisation, selon l'une quelconque des revendications précédentes, dans laquelle la préparation d'oligosaccharides sulfatés est une solution d'oligosaccharides sulfatés comprenant moins de 1% de carrabioses.

10. Utilisation, selon l'une des revendications précédentes, dans laquelle la composition comprend au moins un principe actif amincissant choisi dans le groupe constitué par la caféine, la théophylline, la théobromine, la forskoline, l'acide caféique ou ses sels, l'acide carboxylique caféine ou ses sels, un extrait de racine de *co*/*eus Forskohlii,* un extrait d'écorce de *cecropia obtusa* et un extrait de laitue de mer (*ulva lactuca*).

11. Composition cosmétique amincissante comprenant une préparation d'oligosaccharides sulfatés capable de piéger la spermine et/ou la spermidine, comme principe actif amincissant, éventuellement en association avec un autre principe actif amincissant, tel que la caféine, la théophylline, la théobromine, la forskoline, un extrait de racine de *coleus Forskohlii,* un extrait d'écorce de *cecropia obtusa,* un extrait de laitue de mer (*ulva lactuca*) et/ou un de leurs mélanges.

12. Composition cosmétique selon la revendication 11, dans laquelle il s'agit d'une composition nutraceutique.

13. Utilisation d'une préparation d'oligosaccharides sulfatés telle que définie dans l'une quelconque des revendications 1 à 10, éventuellement en association avec un autre principe actif amincissant, tel que la caféine, la théophylline, la théobromine, la forskoline, un extrait de racine de *coleus Forskohlii,* un extrait d'écorce de *cecropia obtusa,* un extrait de laitue de mer (*ulva lactuca*) et/ou un de leurs mélanges, pour la préparation d'une composition pharmaceutique à effet amincissant, notamment pour lutter contre une surcharge pondérale.

14. Méthode de soin cosmétique non-thérapeutique amincissant comprenant l'application topique ou l'administration orale d'une composition cosmétique selon l'une des revendications 11 ou 12.

15. Composition pharmaceutique pour utilisation dans un traitement thérapeutique pour réduire la quantité de lipides stockés par les adipocytes, par administration ou application topique d'une préparation d'oligosaccharides sulfatés capable de piéger la spermine et/ou la spermidine telle que définie dans l'une quelconque des revendications 1 à 10.

16. Composition comprenant (i) une préparation d'oligosaccharides sulfatés capable de piéger la spermine et/ou la spermidine, notamment telle que définie dans l'une quelconque des revendications 2 à 10, et (ii) au moins un composé du groupe constitué de la caféine, la théophylline, la théobromine, et de la forskoline.

17. Composition selon la revendication 16, dans laquelle (ii) est de la caféine et/ou un de ses sels.

18. Composition selon la revendication 16, dans laquelle (ii) est de la forskoline et/ou un de ses sels.
